# EUROPEAN PATENT SPECIFICATION

(11) **EP 1 985 302 B1**
(45) Date of publication and mention of the grant of the patent: **03.01.2018**
(21) Application number: 07702136.8
(22) Date of filing: 19.01.2007
(51) Int. Cl.: A61K 47/60, A61P 35/00, A61K 38/38, A61K 38/40, C07K 14/78, A61K 38/36

(54) **MEDICAMENT FOR TREATMENT OF TUMOR AND THE USE THEREOF**
ARZNEIMITTEL ZUR TUMORTHERAPIE UND SEINE VERWENDUNG
MÉDICAMENT POUR LE TRAITEMENT DE TUMEURS ET SON UTILISATION

(30) Priority: 20.01.2006 CN 200610011247
(43) Date of publication of application: 29.10.2008
(73) Proprietor: Tsinghua University, Haidian District, Beijing 100084 (CN); Protgen Ltd., Haidian District, 100085 Beijing (CN)
(72) Inventor: LUO, Yongzhang, Beijing 100084 (CN); HAN, Qing, Beijing 100084 (CN); LEI, Qingxin, Beijing 100085 (CN); CHANG, Guodong, Beijing 100085 (CN); FU, Yan, Beijing 100084 (CN)
(74) Representative: Nederlandsch Octrooibureau
(86) International application number: PCT/CN2007/000204
(87) International publication number: WO 2007/082483

(56) References cited:
- WO-A1-90/15628
- WO-A2-01/87925
- WO-A2-2004/020469
- CN-A- 1 518 595
- CN-A- 1 708 513
- US-A1- 2004 110 671
- VERONESE F M: "Peptide and protein PEGylation - a review of problems and solutions", BIOMATERIALS, ELSEVIER SCIENCE PUBLISHERS BV., BARKING, GB, vol. 22, no. 5, 1 March 2001 (2001-03-01), pages 405-417, XP004227886, ISSN: 0142-9612, DOI: 10.1016/S0142-9612(00)00193-9
- ROBERTS M J ET AL: "Chemistry for peptide and protein PEGylation", ADVANCED DRUG DELIVERY REVIEWS, ELSEVIER BV, AMSTERDAM, NL, vol. 54, no. 4, 17 June 2002 (2002-06-17), pages 459-476, XP002293146, ISSN: 0169-409X, DOI: 10.1016/S0169-409X(02)00022-4
- MU J. ET AL.: 'Modification of HCG with Activated mPEG5000 and Study on Its Activity' CHINESE JOURNAL OF BIOLOGICALS vol. 8, no. 2, 1995, pages 53 - 55

### SEQUENCE LISTING

<110> Tsinghua University
   PROTGEN LTD.
<120> Medicament for treatment of tumors and the use thereof
<130> P2007433C
<150> 200610011247.9
   <151> 2006-01-20
<160> 8
<170> PatentIn version 3.3
<210> 1
   <211> 183
   <212> PRT
   <213> Homo sapiens
<400> 1
   His Ser His Arg Asp Phe Gln Pro Val Leu His Leu Val Ala Leu Asn
   1               5                   10                  15
   Ser Pro Leu Ser Gly Gly Met Arg Gly Ile Arg Gly Ala Asp Phe Gln
               20                  25                  30
   Cys Phe Gln Gln Ala Arg Ala Val Gly Leu Ala Gly Thr Phe Arg Ala
           35                  40                  45
   Phe Leu Ser Ser Arg Leu Gln Asp Leu Tyr Ser Ile Val Arg Arg Ala
       50                  55                  60
   Asp Arg Ala Ala Val Pro Ile Val Asn Leu Lys Asp Glu Leu Leu Phe
   65                  70                  75                  80
   Pro Ser Trp Glu Ala Leu Phe Ser Gly Ser Glu Gly Pro Leu Lys Pro
                   85                  90                  95
   Gly Ala Arg Ile Phe Ser Phe Asp Gly Lys Asp Val Leu Arg His Pro
               100                 105                 110
   Thr Trp Pro Gln Lys Ser Val Trp His Gly Ser Asp Pro Asn Gly Arg
           115                 120                 125
   Arg Leu Thr Glu Ser Tyr Cys Glu Thr Trp Arg Thr Glu Ala Pro Ser
       130                 135                 140
   Ala Thr Gly Gln Ala Ser Ser Leu Leu Gly Gly Arg Leu Leu Gly Gln
   145                 150                 155                 160
   Ser Ala Ala Ser Cys His His Ala Tyr Ile Val Leu Cys Ile Glu Asn
                   165                 170                 175
   Ser Phe Met Thr Ala Ser Lys
               180
<210> 2
   <211> 184
   <212> PRT
   <213> Homo sapiens
<400> 2
   Met His Ser His Arg Asp Phe Gln Pro Val Leu His Leu Val Ala Leu
   1               5                   10                  15
   Asn Ser Pro Leu Ser Gly Gly Met Arg Gly Ile Arg Gly Ala Asp Phe
               20                  25                  30
   Gln Cys Phe Gln Gln Ala Arg Ala Val Gly Leu Ala Gly Thr Phe Arg
           35                  40                  45
   Ala Phe Leu Ser Ser Arg Leu Gln Asp Leu Tyr Ser Ile Val Arg Arg
       50                  55                  60
   Ala Asp Arg Ala Ala Val Pro Ile Val Asn Leu Lys Asp Glu Leu Leu
   65                  70                  75                  80
   Phe Pro Ser Trp Glu Ala Leu Phe Ser Gly Ser Glu Gly Pro Leu Lys
                   85                  90                  95
   Pro Gly Ala Arg Ile Phe Ser Phe Asp Gly Lys Asp Val Leu Arg His
               100                 105                 110
   Pro Thr Trp Pro Gln Lys Ser Val Trp His Gly Ser Asp Pro Asn Gly
           115                 120                 125
   Arg Arg Leu Thr Glu Ser Tyr Cys Glu Thr Trp Arg Thr Glu Ala Pro
       130                 135                 140
   Ser Ala Thr Gly Gln Ala Ser Ser Leu Leu Gly Gly Arg Leu Leu Gly
   145                 150                 155                 160
   Gln Ser Ala Ala Ser Cys His His Ala Tyr Ile Val Leu Cys Ile Glu
                   165                 170                 175
   Asn Ser Phe Met Thr Ala Ser Lys
               180
<210> 3
   <211> 44
   <212> PRT
   <213> Homo sapiens
<400> 3
   Phe Gln Pro Val Leu His Leu Val Ala Leu Asn Ser Pro Leu Ser Gly
   1               5                   10                  15
   Gly Met Arg Gly Ile Arg Gly Ala Asp Phe Gln Cys Phe Gln Gln Ala
                   20                  25                  30
   Arg Ala Val Gly Leu Ala Gly Thr Phe Arg Ala Phe
           35                  40
<210> 4
   <211> 45
   <212> PRT
   <213> Homo sapiens
<400> 4
   Tyr Cys Glu Thr Trp Arg Thr Glu Ala Pro Ser Ala Thr Gly Gln Ala
   1               5                   10                  15
   Ser Ser Leu Leu Gly Gly Arg Leu Leu Gly Gln Ser Ala Ala Ser Cys
               20                  25                  30
   His His Ala Tyr Ile Val Leu Cys Ile Glu Asn Ser Phe
           35                  40                  45
<210> 5
   <211> 27
   <212> PRT
   <213> Homo sapiens
<400> 5
   His Ser His Arg Asp Phe Gln Pro Val Leu His Leu Val Ala Leu Asn
   1               5                   10                  15
   Ser Pro Leu Ser Gly Gly Met Arg Gly Ile Arg
               20                  25
<210> 6
   <211> 192
   <212> PRT
   <213> Homo sapiens
<400> 6
   Met Gly Gly Ser His His His His His His Ser His Arg Asp Phe Gln
   1               5                   10                  15
   Pro Val Leu His Leu Val Ala Leu Asn Ser Pro Leu Ser Gly Gly Met
               20                  25                  30
   Arg Gly Ile Arg Gly Ala Asp Phe Gln Cys Phe Gln Gln Ala Arg Ala
           35                  40                  45
   Val Gly Leu Ala Gly Thr Phe Arg Ala Phe Leu Ser Ser Arg Leu Gln
       50                  55                  60
   Asp Leu Tyr Ser Ile Val Arg Arg Ala Asp Arg Ala Ala Val Pro Ile
   65                  70                  75                  80
   Val Asn Leu Lys Asp Glu Leu Leu Phe Pro Ser Trp Glu Ala Leu Phe
   85 90 95
   Ser Gly Ser Glu Gly Pro Leu Lys Pro Gly Ala Arg Ile Phe Ser Phe
   100 105 110
   Asp Gly Lys Asp Val Leu Arg His Pro Thr Trp Pro Gln Lys Ser Val
   115 120 125
   Trp His Gly Ser Asp Pro Asn Gly Arg Arg Leu Thr Glu Ser Tyr Cys
   130 135 140
   Glu Thr Trp Arg Thr Glu Ala Pro Ser Ala Thr Gly Gln Ala Ser Ser
   145 150 155 160
   Leu Leu Gly Gly Arg Leu Leu Gly Gln Ser Ala Ala Ser Cys His His
   165 170 175
   Ala Tyr Ile Val Leu Cys Ile Glu Asn Ser Phe Met Thr Ala Ser Lys
   180 185 190
<210> 7
   <211> 191
   <212> PRT
   <213> Homo sapiens
<400> 7
   Gly Gly Ser His His His His His His Ser His Arg Asp Phe Gln Pro
   1 5 10 15
   Val Leu His Leu Val Ala Leu Asn Ser Pro Leu Ser Gly Gly Met Arg
   20 25 30
   Gly Ile Arg Gly Ala Asp Phe Gln Cys Phe Gln Gln Ala Arg Ala Val
   35 40 45
   Gly Leu Ala Gly Thr Phe Arg Ala Phe Leu Ser Ser Arg Leu Gln Asp
   50 55 60
   Leu Tyr Ser Ile Val Arg Arg Ala Asp Arg Ala Ala Val Pro Ile Val
   65 70 75 80
   Asn Leu Lys Asp Glu Leu Leu Phe Pro Ser Trp Glu Ala Leu Phe Ser
   85 90 95
   Gly Ser Glu Gly Pro Leu Lys Pro Gly Ala Arg Ile Phe Ser Phe Asp
   100 105 110
   Gly Lys Asp Val Leu Arg His Pro Thr Trp Pro Gln Lys Ser Val Trp
   115 120 125
   His Gly Ser Asp Pro Asn Gly Arg Arg Leu Thr Glu Ser Tyr Cys Glu
   130 135 140
   Thr Trp Arg Thr Glu Ala Pro Ser Ala Thr Gly Gln Ala Ser Ser Leu
   145 150 155 160
   Leu Gly Gly Arg Leu Leu Gly Gln Ser Ala Ala Ser Cys His His Ala
   165 170 175
   Tyr Ile Val Leu Cys Ile Glu Asn Ser Phe Met Thr Ala Ser Lys
   180 185 190
<210> 8
   <211> 9
   <212> PRT
   <213> Artificial
<220>
   <223> His-tag
<400> 5

## Description

### Field of the invention

The present invention relates to biologically active and metabolically stable conjugates comprising endostatin and sustained-release formulations thereof and methods of preparing them according to the claims. The present invention also provides pharmaceutical compositions and kits which contain the conjugates or the sustained-release formulation mentioned above. The present invention also relates to the use of the endostatin conjugates, the sustained-release formulations, the pharmaceutical compositions and the kits for preventing, diagnosing, and treating tumors, and the use of the endostatin conjugates for preparing antitumor medicaments.

### Background of the invention

Angiogenesis is the growth of new capillaries from existing blood vessels. The growth and migration of tumor cells depend on angiogenesis, and thus taking tumor vascular endothelial cells as the target of cancer therapy provides a new strategy for cancer treatment. (Folkman J. N Engl J Med 1971; 285:1182-1186).

Endostatin is a 20-kDa cleavage fragment of the C-terminus of collagen XVIII. In 1997, Professor Folkman *et al.* from Harvard University discovered this protein in the culture medium of the hemangioendothelioma cells, which had inhibitory activities on the proliferation, migration of vascular endothelial cells, and the formation of blood vessels *in vivo.* Further experiments revealed that the recombinant endostatin can inhibit the growth and metastasis of various types of tumors in mice, and can even cure the tumor without inducing drug resistance. The mechanism underlying the activities of endostatin is that it suppresses the angiogenesis in tumor tissues by inhibiting the growth of vascular endothelial cells. Consequently, the tumor cannot obtain necessary nutrition and oxygen needed for its growth, which results in growth inhibition or necrosis (Folkman J. et al. Cell 1997; 88:277-285; Folkman J. et al. Nature 1997; 390:404-407). Recombinant human endostatin prepared by gene engineering has been developed into anti-tumor conjugate and shows effective inhibitory effect on tumor growth in clinical trials. In China, it shows outstanding anti-tumor effect in clinical trials on the non-small-cell lung carcinoma.

Compared with chemical drugs, the polypeptide and protein drugs have the advantages of low toxicity/side effects and little drug resistance etc. In order to achieve higher activity, bioavailability, and lower degradation *in vivo,* the protein drugs are usually administrated intravenousely. But in this context, the half-life of low molecular weight protein drugs would be very short, not only because of the degradation, but also the quick elimination via kidney. In blood, if the proteins' hydraulic radiuses are larger than that of albumin or their molecular weights are larger than 66kDa, they can be reserved stably in circulation. However, protein of lower molecular weight will be quickly eliminated from the blood via glomeruli. As such, in order to maintain the effective treatment concentration of low molecular weight proteins in blood, frequent injection or continuous infusion is required. Although treatment in such a way could achieve the therapeutic effects, but it also causes inconveniences and pains to the patients and increases the cost of treatment. Meanwhile, long term administration of some drugs may cause some side effects, for instance, immunological reactions.

As a protein drug, endostatin also has the disadvantages of short half-life and high elimination rate *in vivo.* At present, endostatin is administrated by frequent intravenous injection or infusion, generally daily, for a long term, which means a heavy burden on both psychological and economical aspects for the patients. The purpose of the present invention is to improve metabolism characteristics of endostatin, which would result in higher stability, longer half-life, and even higher curative efficiency *in vivo,* and thereby decrease the frequency and cost of treatment, which could reduce the burden on the patients.

Using high molecular polymer to modify proteins is a common method to change and control the dynamics characteristics of drugs, such as half-life, immunology characteristics, and toxicological characteristic. The high molecular polymer used to modify proteins should have, among others, good water-solubility, good biocompatibility, little or no immunogenicity. The polyethylene glycol is a prevailing protein-modified molecule. The polyethylene glycol has the amphipathic properties, which can be dissolved not only in water, but also in most of the organic solvents. Meanwhile, the polyethylene glycol is non-toxic, non-immunogenic, and highly soluble in water, and thus has been approved as a high molecular polymer for drug preparation by many countries' drug administrations including SFDA of China as well as FDA of the U.S.A.. Coupling the protein with high molecular polymers, for example, the polyethylene glycol, can increase the protein's *in vivo* stability, decrease nonspecific adsorption and antigenicity. Once the conjugate reaches a certain molecular weight, the eliminating rate by kidney can be reduced effectively, which is an effective measure to prolong the half-life of the protein drug *in vivo* (Frokjaer S. et al. Nat. Rev. Drug Discov. 2005 Apr 4(4): 298-306; Harris JM. et al. Nat. Rev. Drug Discov. 2003 Mar 2(3): 214-21). The amino groups initially used as the reaction site in the modification of the polyethylene glycol were mainly α-amino group of N-terminus of the protein, and ε-amino group of the side chain of the lysine residue. The product of the reaction is a protein molecule coupled with one or multiple PEG molecules. The modification of ε-amino group in the side chain of the lysine residue may generate modified isomerides due to the non-specific reaction sites.

Recently, aiming at the difference of isoelectric points between α-amino group of N-terminus of the protein and ε-amino group of the side chain of the lysine residue, polyethylene glycol modifying agents have been developed which specifically modify N-terminus of the protein and as a result, it is possible to obtain uniform modification products with modification at the identical site. Another protein site for modification with the polyethylene glycol is the mercapto group of the cysteine residue. Generally, the number of the mercapto groups is fewer than that of the amino groups in a protein, and thus modification of the mercapto groups is more specific. Using genetic engineering techniques, now it is possible to introduce a cysteine at any position of a protein to serve as a specific modification site. But introducing a cysteine as the modification site also has certain limitations because, for those proteins that already contain cysteine residues, this may cause mispairing of disulfide bonds or make it difficult for such proteins to renature. In addition, the carboxyl group of the protein is also frequently used as a site for modification (Veronese FM et al. Drug Discov. Today. 2005 Nov 1;10(21):1451-8.). Modification techniques with polyethylene glycol have been successfully used in a plurality of protein drugs, including PEG-asparaginase (Graham ML Adv. Drug Deliv. Rev. 55, 1293-1302 Avramis Vassilios I. et al. WO9939732 and US6689762), PEG-adenosine deaminase (Levy Y et al. J. Pediatr. 113, 312-317; Davis S et al. ClinExp. Immunol. 46: 649-652; Hershfield MS et al. N Engl J Med 316 : 589-596), PEG-interferons such as PEG-interferon α2a and PEG-interferon α2b etc. (Bailon P et al. C. Bioconjug. Chem. 12, 195-202, Wang YS et al. Adv. Drug Deliv. Rev. 54, 547-570, Meng Xiantai et al. WO2005077421, Van Vlasselaer Peter et al. WO2004076474, Ballon Pascal Sebastlan et al. US2004030101, Karasiewicz Robert et al. EP0593868) etc.

Other representative high molecular modifying agents include glucan, the polysucrose, starch, polyalanine, copolymer of ethanedioic acid and the malonic acid, carboxymethyl cellulose, polyvinylpyrrolidone, poly 1,3-dioxolane, copolymer of ethylene and maleic hydrazide, poly sialic acid, cyclodextrin, etc.

Another method of prolonging the half-life of a protein drug is to couple or fuse it with a blood protein or a fragment thereof used as a carrier to increase the molecular weight of the protein drug. For example, Fc fragment of immunoglobulin may be coupled with the target protein to prolong the half-life of the latter. For example, this strategy has been used in the Notch 1 receptor protein (Kitajewsky Jan et al. WO2005111072), erythropoietin (EPO) (Gillies Stephen D et al. WO2005063808), human somatropin (Kim Young Min et al. WO2005047337), etc. The plasma albumin is another commonly-used coupling carrier, which is used in the proteins such as some antibiotics, anti-inflammatory agents, and anti-oxidants (Ezrin Alan M et al. WO0117568, Otagiri Masaki et al. EP1571212), etc.

In addition to directly coupling the protein drug with some blood protein carriers *in vitro,* it is also possible to modify the drug protein *in vitro* to provide the drug protein with chemical reaction activity or high affinity for some molecules *in vivo,* so that the drug protein can react with some components of the blood upon entering into the body to form macromolecules or compounds with longer half-life. One example is the antiviral small peptide anti-RSV modified with an active group carrying maleimide, which can react with the mercapto groups of the blood proteins or the cell surface proteins (Bridon Dominique P et al. WO0069902). Another example is to introduce fatty acids to the amino acid residues on the surface of the protein by acylation reaction so as to increase the affinity of the protein to the albumin *in vivo.* Upon adminstered into the blood, the protein can form a larger complex with albumin and thereby the half-life of the protein is prolonged. The method has been used to produce long-acting insulins (Kurtzhals P et al. Biochem. J. (1995) 312, 725-731; Frokjaer S et al. Nat Rev Drug Discov. 2005 Apr;4(4):298-306).

Sustained release formulation is another approach of prolonging the *in vivo* half-life of a protein drug. The protein drug is placed in a pharmaceutical carrier to allow the protein to be released from the carrier slowly, and thereby a stable *in vivo* drug concentration is maintained. The commonly used sustained release formulations comprise hydrogel, microcapsule, microballoon, micro-osmotic pump, liposome etc. (Peppas NA et al. Eur. J. Pharm. Biopharm. 50, 27-46 (2000); Packhaeuser CB et al. Eur. J. Pharm. Biopharm. 58, 445-455 (2004); Metselaar JM et al. Mini Rev. Med. Chem. 4, 319-329 (2002)). Liposome is an ultramicroscopic particle in the form of a hollow sphere with a bilayer membrane structure. The bilayer membrane is composed of amphipathic molecules, most of which are phosphatides, and forms a hydrophilic inner chamber. The hydrophilic protein drug is encapsulated in the inner chamber of the liposome and thus can be slowly released *in vivo* to maintain the concentration of the protein drug and prolong the half-life. Examples are nerve growth factor (Hou Xinpu et al. CN1616087) and hemoglobin (Farmer Martha C et al. US4911929) etc.

WO 01/87925 generally relates to methods for obtaining refolded, soluble forms of proteins having one or more free cystein residues and which are expressed by a host cell in an insoluble or aggregated form. As an example of such proteins, endostatin was mentioned and tested in WO 01/87925.

### Summary of the invention

Subject-matter which is not encompassed by the scope of the claims does not form part of the presently claimed invention. The present disclosure provides a conjugate formed by a modifying agent and endostatin, wherein the modifying agent is capable of prolonging the half-life of endostatin according to the claims. The modifying agent used in the present disclosure may be attached to endostatin via covalent bond. These modifying agents are macromolecules which include, for example, polyethylene glycol with an average molecular weight in the range from 1,000 to 100,000 Daltons, preferably 5,000 to 40,000 Daltons.

The present invention relates to a conjugate formed by coupling one polyethylene glycol molecule to one endostatin molecule, wherein the polyethylene glycol molecule is coupled to endostatin at the N-terminal α-amino group of endostatin, and the polyethylene glycol has an average molecular weight from 5 kDa to 40 kDa, and wherein the conjugate has enhanced biological activities as compared to the corresponding non-modified endostatin molecule.

In the present invention, the conjugate is an endostatin molecule coupled with one polyethylene glycol molecule.

In the conjugate of the present disclosure formed by polyethylene glycol and endostatin, the coupling site is the N-terminal α-amino group, ε-amino group of the side chain of a lysine residue, mercapto group of the side chain of a cysteine residue, carboxyl group of the side chain of an aspartate residue, carboxyl group of the side chain of a glutamate residue in endostatin or any combination thereof Preferably, the coupling site is N-terminal α-amino group of endostatin.

In one embodiment of the conjugate of the present disclosure, the endostatin molecule is coupled with one or more polyethylene glycol molecules at the N-terminal α-amino group of said endostatin molecule or the ε-amino group on the side chain of a lysine residue corresponding to the 75th, 95th, 106th, 117th or 183rd lysine residue of the wild-type endostatin or any combination thereof.

In one embodiment of the conjugate of the present disclosure, the endostatin molecule is coupled with one or more polyethylene glycol molecules at mercapto groups on the side chains of additional cysteine residues, wherein the additional cysteine residues are introduced by adding cysteine residues, or peptide chains containing cysteine residues, to the N-terminus, C-terminus, or the internal region of said endostatin molecule.

In one embodiment of the conjugate of the present disclosure, the endostatin molecule is coupled with one or more polyethylene glycol molecules at the carboxyl groups on the side chains of aspartate or glutamate residues in said endostatin molecule.

Proteins that can be used in the conjugate of the present disclosure are selected from a group consisting of albumin, immunoglobulin, thyroxin-binding protein, transthyretin, transferrin, fibrinogen and fragments thereof.

In one embodiment of the present disclosure, the conjugate formed by coupling one endostatin molecule to one or more albumins. The conjugate may be obtained via chemical modification, fusion expression, or other methods. Preferably, the albumin is human serum albumin or its fragment. In another embodiment of the present disclosure, the conjugate is formed by coupling one endostatin molecule to one or more immunoglobulin Fc fragments. The conjugate may be obtained either via chemical modification, or via fusion expression, or via other methods. Preferably, the Fc fragment is a fragment of the Fc region of human immunoglobulin IgG.

The conjugate of the present disclosure also includes endostatin modified with small molecules or small peptides or other compounds, wherein the modified endostatin is capable of reacting or binding with other molecules or components *in vivo,* allowing modified endostatin to form larger complex with other molecules or components *in vivo.* The modified endostatin contains a reactive group capable of forming covalent bond with an amino, hydroxyl, or mercapto group of a blood component. The reactive group can be, for example, maleimide, which may react with blood components such as the mercapto group of albumin. In this way, the conjugate of the present disclosure will have strong affinity to some blood components such
as albumin or immunoglobulin, allowing the formation of larger complex.

The conjugate of the present disclosure also includes endostatin modified with other molecules or small peptides. For example, the endostatin can be a glycosylated, phosphorylated or acylated product, in which modifying sites are amino acid residues existing in the original protein sequence or amino acid residues generated by mutation.

The conjugate of the present invention formed by a modifying agent and endostatin may also be a conjugate formed by coupling a modifying agent to endostatin via non-covalent interaction. The modifying agents may be proteins, small molecules or other compounds.

The conjugate of the present invention may form a sustained-release formulation with a bio-compatible carrier. The sustained-release formulation may be in a form selected from the group consisting of microcapsule, hydrogel, microsphere, micro-osmotic pump or liposome.

The endostatin contained in the conjugate or sustained-release formulation of the present invention may be of human, murine or other mammals origin.

In a specific embodiment of the present invention, the endostatin contained in the conjugate or sustained-release formulation is a wild-type human endostatin, the naturally occurring form of which has a sequence as shown in SEQ ID NO.1. When the endostatin contained in the conjugate or sustained-release formulation is one expressed in *E. coli,* the wild-type recombinant human endostatin has a sequence as shown in SEQ ID NO.2. The N-terminal Met sometimes may be deleted when expressed in *E. coli,* thus the corresponding recombinant human endostatin has a sequence as shown in SEQ ID NO.1.

In the conjugate or sustained-release formulation of the present invention, endostatin may be an active fragment, mutant, derivative, isomer of endostatin or a combination thereof. Preferably, in the conjugate or sustained-release formulation of the present invention, the fragment of endostatin is a peptide having a sequence as shown in SEQ ID NO.3, SEQ ID NO.4 or SEQ ID NO.5. In one embodiment of the present invention, the derivative of endostatin is formed by adding a peptide of 1 to 15 amino acids in length to N-terminus or C-terminus of the wild-type endostatin. For example, in the conjugate or sustained-release formulation of the present invention, the derivative of endostatin is formed by adding a nine-amino acid His-tag MGGSHHHHH to N-terminus of the wild-type human endostatin, and thus the derivative has a sequence as shown in SEQ ID NO.6. When the above derivative is expressed in *E. coli,* the N-terminal Met sometimes will be partially deleted, and thus the corresponding derivative may have a sequence as shown in SEQ ID NO.7.

The present invention also provides a pharmaceutical composition comprising a conjugate or sustained-release formulation of the present invention and a pharmaceutically acceptable carrier. The present invention also provides kits comprising the conjugate, composition or sustained-release formulation of the present invention and instruction for the usage thereof.

The present invention also provide method of preparing an endostatin-containing conjugate as mentioned above comprising the steps of mixing activated polyethylene glycol with endostatin and allowing reaction under proper conditions including solution, temperature, pH, and reaction molar ratio. Preferably, the pH used in the method is pH 3 to pH 10. In the method mentioned above, the conjugate product may be purified, for example, via cation exchange column or gel filtration.

The present invention also provides the use of an endostatin-containing conjugate, sustained-release formulation, pharmaceutical composition or kit as mentioned above in the prevention, diagnosis and treatment of tumor and in the prevention, diagnosis and treatment of other diseases characterized by human tissue or organ lesions caused by abnormal neovascularization.

The present invention also provides a method for prolonging the *in vivo* half-life of endostatin comprising the step of forming a conjugate between endostatin and a modifying agent capable of prolonging *in vivo* half-life of endostatin, or comprising the step of providing a sustained-release formulation comprising endostatin or endostatin containing conjugate and a bio-compatible substance.

These and other aspects of the present invention will be elucidated in the following detailed description of the invention. It should be understood that the above general description and the following detailed description as well as the examples are merely for the purpose of illustration, and are not intended to limit the scope of present invention.

### Brief description of the drawings

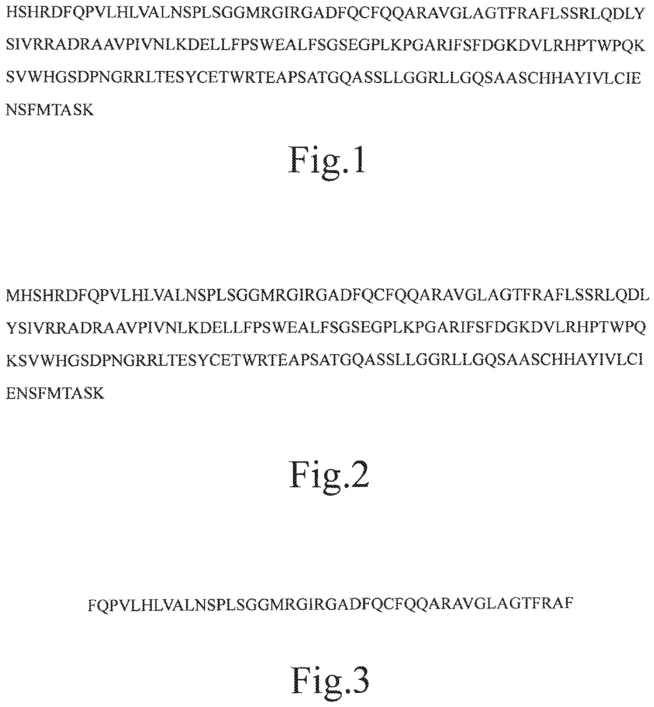

FIG. 1 depicts the amino acid sequence of wild-type human endostatin (SEQ ID NO.1).
FIG. 2 depicts the amino acid sequence of a wild-type human endostatin (SEQ ID NO.2) expressed in a strain of *E. coli.* The Met residue in the N-terminal may sometimes be deleted. In this case, the sequence of N-terminal Met-deleted endostatin will have the sequence of SEQ ID NO.1.
FIG. 3 depicts the amino acid sequence of a biologically active fragment of endostatin (SEQ ID NO.3).

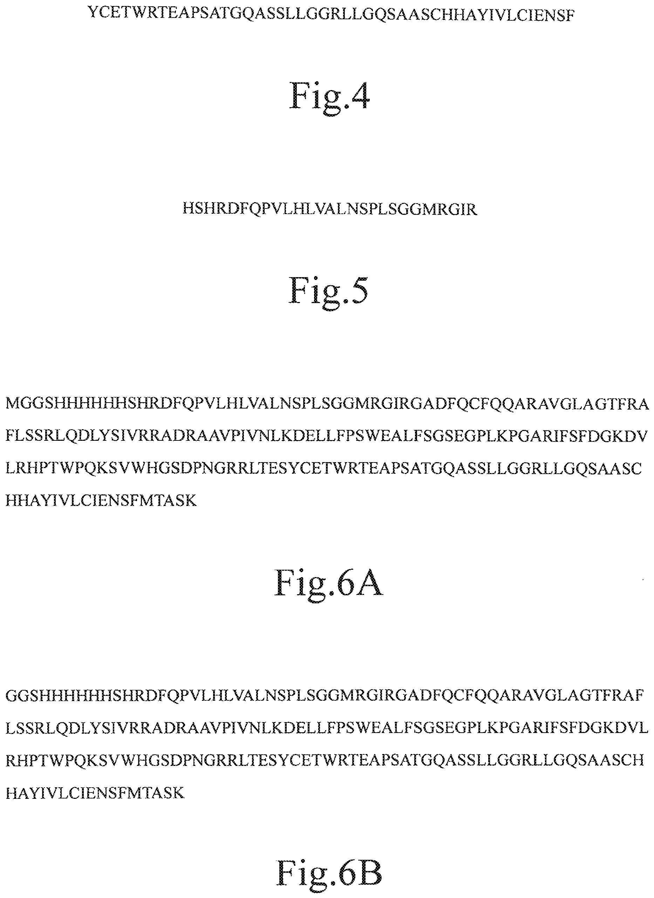

FIG. 4 depicts the amino acid sequence of another biologically active fragment of endostatin (SEQ ID NO.4).
FIG. 5 depicts the amino acid sequence of another biologically active fragment of endostatin (SEQ ID NO.5).
FIG. 6 depicts the amino acid sequence of a biologically active endostatin with additional amino acids at the N-terminus (SEQ ID NO.6, FIG. 6A). N-terminal Met may sometimes be partially deleted after expression. In this case, the N-terminal Met-deleted endostatin will have an amino acid sequence of SEQ ID NO.7 (FIG. 6B).

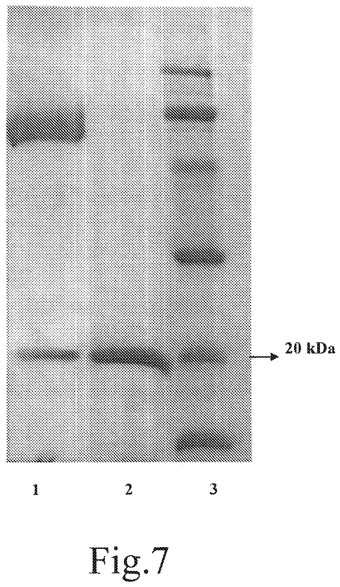

FIG. 7 shows the coupling of 20 kDa polyethylene glycol to the N-terminus of recombinant human endostatin having a sequence of SEQ ID NO.2. The solutions of pre- and post-reaction are subjected to SDS-PAGE detection, in which, Lane 1, 20 kDa polyethylene glycol modified endostatin; Lane 2, unmodified endostatin; Lane 3, protein marker.

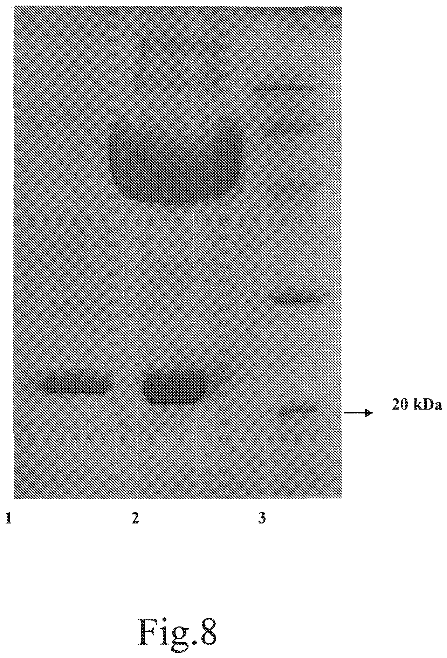

FIG. 8 shows the coupling of 20 kDa polyethylene glycol to the N-terminus of recombinant human endostatin having a sequence of SEQ ID NO.6. The solutions of pre- and post-modification are subjected to SDS-PAGE detection, in which, Lane 1, unmodified endostatin; Lane 2, 20 kDa polyethylene glycol modified endostatin; Lane 3, protein marker.

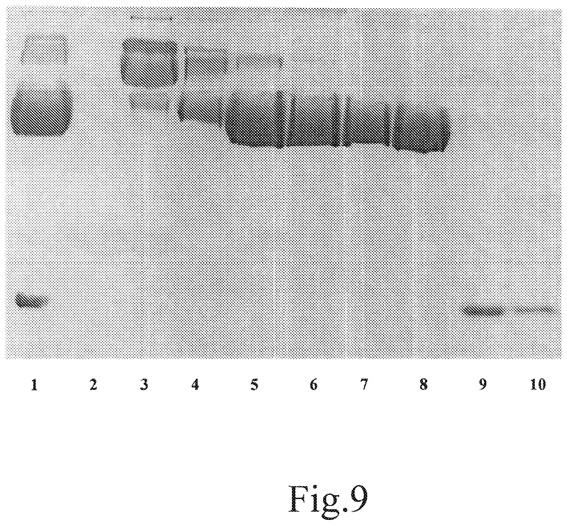

FIG. 9 shows the cation-exchange chromatography SP purification of recombinant human endostatin having a sequence of SEQ ID NO.2, the N-terminus of which is modified with 20 kDa polyethylene glycol. The proteins are bound to the cation-exchange column SP, then eluted with solution of a sodium chloride gradient, finally subjected to reduced SDS-PAGE detection. Lane 1, before purification; Lane 2, flow through; Lanes 3-10, collected elution fractions.

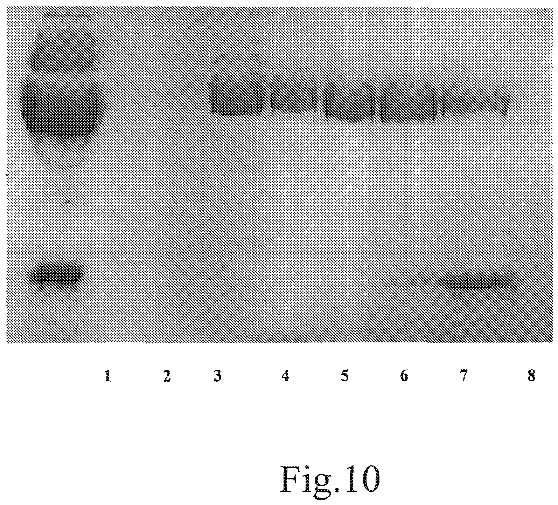

FIG. 10 shows the cation-exchange chromatography SP purification of recombinant human endostatin having a sequence of SEQ ID NO.6, the N-terminus of which is modified with 20 kDa polyethylene glycol. The proteins are bound to the cation-exchange column SP, then eluted with solution of a sodium chloride gradient, finally subjected to reduced SDS-PAGE detection. Lane 1, before purification; Lane 2, flow through; Lanes 3-8, collected elution fractions.

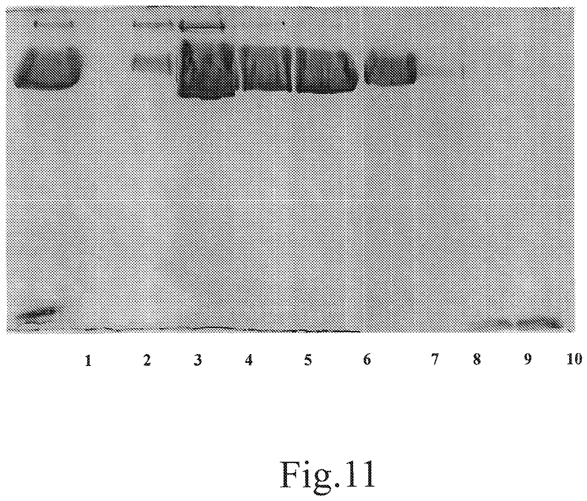

FIG. 11 shows the cation-exchange chromatography SP purification of recombinant human endostatin having a sequence of SEQ ID NO.2, the N-terminus of which is modified with 40 kDa polyethylene glycol. The proteins are bound to the cation-exchange column SP, then eluted with solution of a sodium chloride gradient, finally subjected to reduced SDS-PAGE detection. Lane 1, before purification; Lane 2, flow through; Lanes 3-10, collected elution fractions.

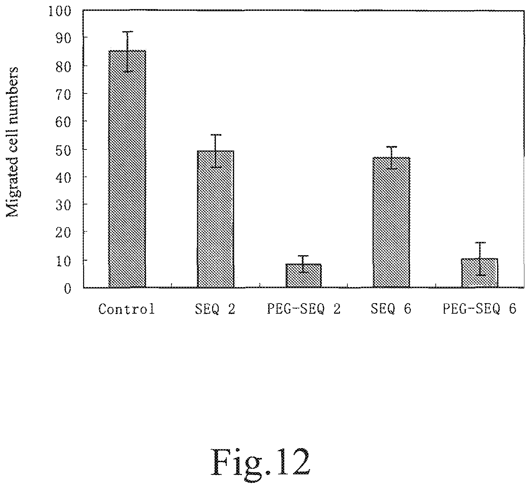

FIG. 12 shows the activity of the recombinant human endostatin N-terminus coupled with 20 kDa polyethylene glycol to inhibit the migration of vascular endothelial cells. SEQ 2: endostatin of SEQ ID NO.2; PEG-SEQ 2: 20 kDa polyethylene glycol modified endostatin of SEQ ID NO.2; SEQ 6: endostatin of SEQ ID NO.6; PEG-SEQ 6: 20kDa polyethylene glycol modified endostatin of SEQ ID NO.6.

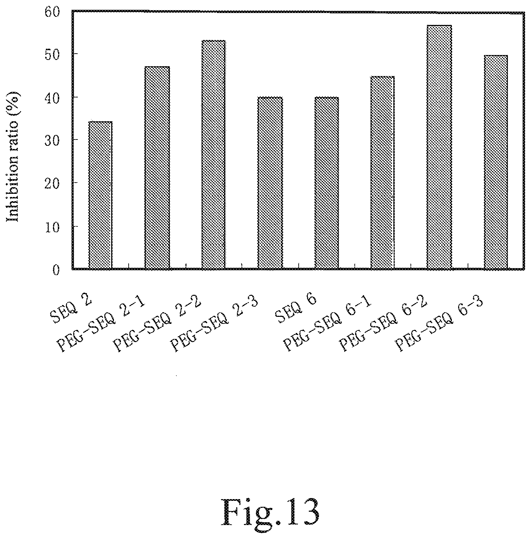

FIG. 13 shows the activity of the recombinant human endostatin N-terminus coupled with 20 kDa polyethylene glycol to inhibit sarcoma S180 in mouse. SEQ 2: daily administration with endostatin of SEQ ID NO.2; PEG-SEQ 2-1: daily administration with 20 kDa polyethylene glycol modified endostatin of SEQ ID NO.2; PEG-SEQ 2-2: administration every other day with 20 kDa polyethylene glycol modified endostatin of SEQ ID NO.2; PEG-SEQ 2-3: administration every three days with 20 kDa polyethylene glycol modified endostatin of SEQ ID NO.2; SEQ 6: daily administration with endostatin of SEQ ID NO.6; PEG-SEQ 6-1: daily administration with 20 kDa polyethylene glycol modified endostatin of SEQ ID NO.6; PEG-SEQ 6-2: administration every other day with 20 kDa polyethylene glycol modified endostatin of SEQ ID NO.6; PEG-SEQ 6-3: administration every three days with 20 kDa polyethylene glycol modified endostatin of SEQ ID NO.6.

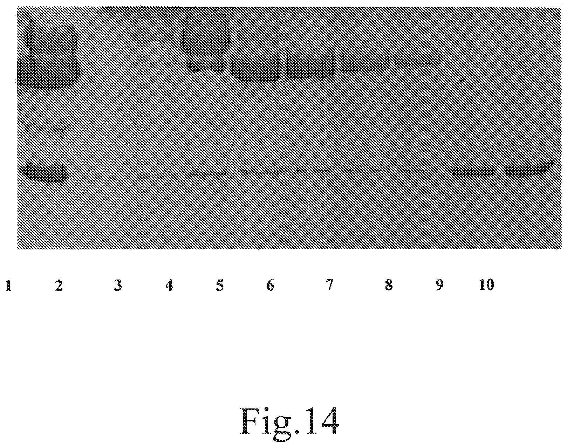

FIG. 14 shows the gel filtration purification of the recombinant human endostatin with a sequence of SEQ ID NO.6, which is modified with 20 kDa polyethylene glycol at the ε-amino group of the side-chain of tyrosine residue. Lane 1, loaded sample; Lanes 2-10, fractionated elutions collected.

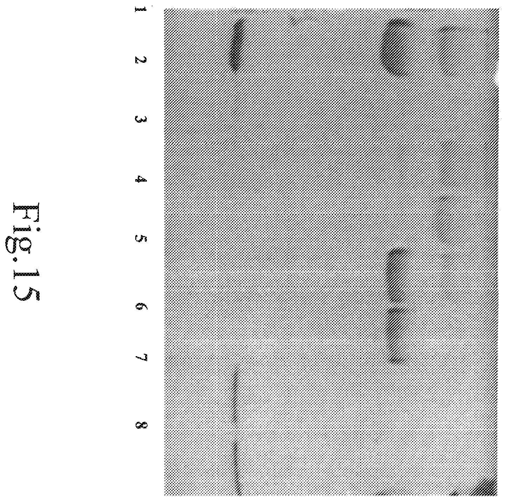

FIG. 15 shows the cation-exchange chromatography CM purification of the recombinant human endostatin with a sequence of SEQ ID NO.6, which is modified with 20 kDa polyethylene glycol at the ε-amino group of the side-chain of tyrosine residue. Lane 1, loaded sample; Lane 2, flow through; Lanes 3-8, fractionated elutions collected.

### Detailed description of the invention

The present invention provides an endostatin product which has a longer half-life. It shows metabolic stability and longer half-life *in vivo* than unmodified endostatin, while maintaining the activity in preventing new blood vessel formation. Preferably, the endostatin used in this invention is human endostatin.

One embodiment of the present invention relates to a conjugate formed by a modifying agent and endostatin. The term "conjugate" in this invention refers to a modified endostatin, in which the endostatin and a modifying agent are linked by either a covalent bond or a non-covalent bond to form a stable complex. Therefore, the "modification" can be achieved via either a covalent or non-covalent linkage. The modifying agent can be either chemically coupled to the endostatin or expressed as a part of a fusion with endostatin. In a preferred embodiment, the modification is site specific and will not influence the bioactivity of endostatin, e.g., binding to its receptors. This modified product maintains the antiangiogenic activity and shows higher metabolic stability and longer half-life in blood than the unmodified endostatin, and therefore it can be used as an antiangiogenic or anti-tumor conjugate. The modifying agent can be one or more macromolecule polymers, proteins or its fragments, peptides or other chemical compounds, which is biocompatible and able to increase the half-life of endostatin.

The macromolecule polymers as mentioned above can either have its own bioactivity or not. The suitable macromolecule polymers include, but not limited to, polyethylene alcohols, polyether compounds, polyvinylpyrrolidone, poly amino acids, copolymer of Divinyl ether and maleic anhydride, N-(2-Hydroxypropyl)-methacrylamide, polysaccharide, polyoxyethylated polyol, heparin or its fragment, poly-alkyl-ethylene glycol and its derivatives, copolymers of poly-alkyl-ethylene glycol and its derivatives, poly(vinyl ethyl ether), a,P-Poly ((2-hydroxyethyl)-DL-aspartamide, polycarboxylates, poly oxyethylene-oxymethylenes, polyacryloyl morpholines, copolymer of amino compounds and oxidative olefin, poly hyaluronic acid, polyoxiranes, copolymer of ethanedioic acid and malonic acid, poly 1,3-dioxolane, ethylene and maleic hydrazide copolymer, poly sialic acid, cyclodextrin and etc.

The polyethylene alcohols as mentioned above include, but not limited to, polyethylene glycol (monomethoxy polyethylene glycol, monohydroxy polyethylene glycol), polyvinyl alcohol, polyallyl alcohol, polybutene alcohol and their derivatives. In a preferred embodiment, the modifying agent is polyethylene glycol. In a more preferred embodiment, polyethylene glycol is monomethoxy polyethylene glycol.

The polyethers as mentioned above include, but not limited to, poly alkylene glycol (HO((CH₂)ₓO)ₙH), polypropylene glycol, polyoxyethylene (HO((CH₂)₂O)ₙH), polyvinyl alcohol ((CH₂CHOH)ₙ) and derivatives thereof.

The poly amino acids as mentioned above include, but not limited to, polymers of one type of aminoacid, copolymers of two or more types of amino acids, for example, polyalanine.

The polysaccharides as mentioned above include, but not limited to, glucosan and its derivatives, for example dextran sulfate, cellulose and its derivatives (including methyl cellulose and carboxymethyl cellulose), starch and its derivatives, polysucrose and etc.

The present invention relates to a conjugate formed by coupling one polyethylene glycol molecule to one endostatin molecule, wherein the polyethylene glycol molecule is coupled to endostatin at the N-terminal α-amino group of endostatin, and the polyethylene glycol has an average molecular weight from 5 kDa to 40 kDa, and wherein the conjugate has enhanced biological activities as compared to the corresponding non-modified endostatin molecule.

A preferred protein molecule used as a modifying carrier of the invention is a protein naturally occurring or a fragment thereof, which includes, but not limited to, thyroxine-binding protein, transthyretin, transferrin, fibrinogen, immunoglobulin, albumin and fragments thereof. Preferred carrier proteins are human proteins. A fragment of a protein as mentioned above refers to a part of the protein that still has the activity to serve as a carrier protein.

Endostatin is directly or indirectly linked to the modifying agent via a covalent bond. Direct linking means that one amino acid of endostatin is directly linked to one amino acid of the carrier protein, via peptide bond or a disulfide bridge. Indirect linking means that endostatin is linked to the carrier protein via one or more chemical groups.

There have been some reports on modifying endostatin with polyethylene glycol. Guoying Mou (2005) and Shenglin Yang (2005) reported modifying endostatin with PEG molecules having an average molecule weight of 6 kDa or 10kDa, and the modifying agents are linked to ε-amino groups on the side chains of lysine residues, and thus the product is a mixture of multi-sites modification products. Although the product can be purified by molecular sieve or ion-exchange chromatography to obtain a product of certain range of molecule weight, there are still some drawbacks, such as the inhomogeneity in the modification sites and in the protein conformation, due to the reactive properties of such modifying agents. Moreover, the multi-sites modification of a protein will cause some change of the conformation of internal amino acids, resulting in a decrease of the activity of the protein. For a biological product, the homogeneity of the product is very important for decreasing the toxicity as well as the immunogenicity. In addition, for a mixture, it is also different to ensure the same quality and the stability of efficacy between different batches. Therefore, it is an important issue and a problem to be solved in drug design and preparation how to ensure the consistency of the ingredients, the homogeneity of conformation, and the stability of the activity.

In one embodiment of the present invention, the polyethylene glycol (PEG)-modified endostatin is characterized in that one endostatin molecule is covalently coupled to one PEG molecule. The coupling site of endostatin is the N-terminal α-amino group. The polyethylene glycol used for coupling may be either linear or branched, with an average molecular weight of from 5,000 to 40,000 Daltons. As used herein, the molecular weight of polyethylene glycol refers to the average molecular weight.

Although PEG modification may improve the pharmacokinetic characteristics of the drug, such modification, however, may also reduce the biological activities of a macromolecule drug. The main reason for this is that different modification sites may result in different structural changes and may shield the active sites of the drug (Veronese F., et al. Drug Discovery Today, 10, 21, 2005). For example, after modification with different PEGs, the activity of differently modified Interferon β-1b decreases, and the highest activity of the modified molecule only reached a level corresponding to 71% of the activity of the unmodified, and some modified proteins almost showed no activity at all. So, although PEG modification changes the pharmacokinetic characteristics of a drug, it may also impair the activity of the drug, and consequently. Therefore, both sides are taken into account when selecting the modification process and modifying agents, so as to achieve the optimal overall results *in vivo.* In one embodiment of the present invention, an N-terminal specific Pegylating reagent (mPEG-ButyrALD, Nektar) is used to modify the recombinant Human endostatin. This gives rise to a product which is formed by coupling one PEG molecule to one recombinant endostatin at the N-terminal α-amino group of the protein. It was reported that the Interferon β-1b modified by using the same N-terminal Pegylation reagent only showed 70% of the activity of the unmodified protein in an *in vitro* test (Filpula D., et al. Bioconjugate Chem. 2006, 17, 618-630). In the present invention, however, it is surprisingly found that efficacy of N-terminal modified endostatin reached two times or more that of unmodified protein in inhibiting endothelial cell migration, indicating a prominent increase in the biological activity of endostatin after modification, which has not been reported yet. However, it was reported by G Mou et al (2005) that multiple sites modification of endostatin resulted in a decreased activity in an *in vitro* test. This implies a much better *in vitro* activity of the product of the present invention as compared to the modified endostatin in relevant literatures. On the other hand, as shown in structure analysis, a zinc binding site was found in the N-terminus of human wild-type endostatin, which is consisting of the 1st, 3rd, 11th, and 76th amino acids. It has been reported that these sites are critical for the stability and activity of human endostatin, and form the binding site of human endostatin through which endostatin binds to its receptor and exerts its activity (Ding, Y. H., Proc. Natl. Acad. Sci. U.S.A. 95, 10443-10448; Hohenester, E., EMBO J. 17, 1656-1664; Boehm, T., Biochem. Biophys. Res. Commun. 252, 190-194; Ricard-Blum, S., J. Biol. Chem. 279, 2927-2936; Tjin Tham Sjin, R. M., Cancer Res. 65(9): 3656-63). As PEG has a large molecular weight and large spatial hindrance, it has been proposed that the modification to the protein should not take place at active sites. Otherwise this may greatly destroy the protein structure, which in turn which may impair the activity of the protein. However, the product of the present invention not only avoids activity loss, but exhibits an unexpectedly higher activity.

In one embodiment of the present invention, endostatin is coupled to a protein or peptide, characterized in that one endostatin molecule is directly or indirectly linked to one or more proteins or peptides. In the case that endostatin is coupled to more carrier proteins, the selected carrier proteins can be either the same or different. Preferably, the carrier proteins are naturally occurring proteins or fragments thereof. In a preferred embodiment, the carrier protein selected for modification is human serum albumin or its fragments. In another preferred embodiment, the carrier protein is an Fc fragment of human immunoglobulin IgG.

In another embodiment of the present invention, the modifying agent is a small molecule or a small peptide or other compound, which enables the conjugate comprising endostatin to react with or have high affinity to certain components in the blood. In a specific embodiment, the product has a reactively active group, capable of reacting with amino, carboxyl or mercapto group to form a covalent bond. In another specific embodiment, the active group of the product is maleimide, which is capable of reacting with a mercapto group of a blood component, such as albumin. In another specific embodiment, the product has strong affinity to some blood components such as albumin or immunoglobulin, allowing the formation of larger complex.

In another embodiment of the present invention, the endostatin modified with a small molecule or a small peptide may be glycosylated, phosphorylated or acylated. The modification sites may be amino acid residues originally contained the protein or amino acid residues generated via mutation. Although the molecular weight of the conjugate is not very large, the half-life of the conjugate becomes longer since the modification changes the characteristics of endostatin.

In another embodiment of the present invention, the conjugate comprising endostatin is a conjugate formed via non-covalent interaction between endostatin and a modifying agent such as protein and small molecule. This conjugate has the activity to inhibit angiogenesis and has longer *in vivo* half-life than endostatin.

Another embodiment of the present invention relates to a sustained-release formulation comprising endostatin. The sustained-release formulation is consisting of endostatin or any of the aforementioned conjugate and a biocompatible substance. Endostatin in this composition still has biological activity, and at the same time, its *in vivo* half-life is prolonged because of the carrier which changes the pharmacokinetic characteristics of the drug. The sustained-release formulation can be, but not limited to, a microcapsule, hydrogel, microbeads, micro-osmotic pump and liposome, et al.

The present invention also provides a pharmaceutical composition comprising the aforementioned endostatin-containing conjugate or sustained-release formulation and a pharmaceutically acceptable carrier. Furthermore, the present invention also provides a kit comprising the aforementioned endostatin-containing conjugate or sustained-release formulation and an instruction for use.

The pharmaceutically acceptable carriers used in this invention include carriers, excipients, or stabilizers, which are nontoxic to the cells or mammals to be contacted at selected doses and concentrations. A commonly used physiologically acceptable carrier is an aqueous pH buffer solution. Examples of physiologically acceptable carriers include solutions such as phosphate buffer solution, citrate buffer solution and other organic acid buffer solution; anti-oxidants including ascorbic acid; polypeptides with low molecular weight (no more than 10 residues); proteins such as serum albumin, glutin or immunoglobulin; hydrophilic polymers such as polyvinylpyrrolidone; amino acids such as glycine, glutamine, asparagine, arginine or lysine; monosaccharide, disaccharide and other carbohydrates including glucose, mannose or dextrin; chelating agents such as EDTA; sugar alcohol such as mannitol or sorbitol; salt-forming counter ion such as sodium ion; and/or nonionic surfactant such as TWEEN®, PEG and PLURONICS®. Preferably, the excipients are sterilized and usually free of undesired substance. These compositions can be sterilized by routine sterilizing techniques.

The present invention also relates to methods of preparing the aforementioned products of endostatin. Specifically, the present invention provides a method of preparing a PEGylated endostatin comprising mixing the activated PEG with endostatin, allowing reaction under proper conditions including solution, temperature, pH, and reaction molar ratio, and purifying the coupled product by using cation-exchange column or molecular sieve. Preferably, the pH of this reaction is within 3 to 10.

The present invention also provides the use of the aforementioned endostatin-containing conjugate, sustained-release formulation, pharmaceutical composition or kit in the prevention, diagnosis and treatment of tumor. The tumors that are suitable to be treated with the method of the invention include, but not limited to, lung cancer, neuroendocrine tumor, colon cancer, bone cancer, liver cancer, gastric cancer, pancreatic cancer, oral cancer, breast cancer, prostate cancer, lymphoma, esophagus cancer, oral cancer, nasopharyngeal carcinoma, cervical cancer, sarcoma, renal carcinoma, biliary cancer, malignant melanoma, et al.

The present invention also provides the use of the aforementioned endostatin-containing conjugate, pharmaceutical composition, or sustained-release formulation in preparation of an anti-tumor medicament.

The present invention also provides the use of the aforementioned endostatin-containing conjugate, sustained-release formulation, pharmaceutical composition, or kit for the prevention, diagnosis, or treatment of a disease characterized by, but not limited to, tissue or organ lesions caused by abnormal neovascularization, or in preparation of a medicament for the treatment of such a disease.

According to the present invention, the administration route is selected from the group consisting of intravenous injection, intravenous infusion, subcutaneous injection, intramuscular injection, intraperitoneal injection, percutaneous absorption, subcutaneous embedment, liver arterial injection, oral administration, nasal administration, oral mucosa administration, ocular administration, rectal administration, vaginal delivery, or other clinically acceptable routes.

The present invention also provides a method for prolonging the half-life of endostatin, comprising the step of forming a conjugate between endostatin and a modifying agent capable of prolonging *in vivo* half-life of endostatin and the step of preparing a sustained-release formulation containing endostatin.

Our experimental data show that, a product of the present invention, i.e., a conjugate formed by coupling PEG to the N-terminus of endostatin, has the activity of inhibiting the migration of endothelial cell proliferation and tumor growth in mice. This conjugate showed significantly higher activity as compared with non-modified endostatin. Furthermore, pharmacokinetic study revealed that the *in vivo* metabolism of this modified product was effectively slowed down, and its half-life was prolonged.

Unless otherwise specified, the endostatin as used herein can be of human, murine or other mammals origin. In a preferred embodiment, the endostatin is human endostatin. Unless otherwise specified, the endostatin as used herein can be a wild-type endostatin, i.e., the naturally occurring form; or a biologically active mutant, fragment, isomer, or derivative thereof, or their combinations. Endostatin can be obtained upon expression in animal cells, or can be obtained by fermentation and further purification from yeasts or *E. coli.* The amino acid sequence of the wild-type human endostatin expressed in animal cells is shown in SEQ ID NO.1. The recombinant human endostatin expressed in yeasts may either have an amino acid sequence as shown in SEQ ID NO.1, or have the sequence of SEQ ID NO.1 with no more than 10 amino acids added to or deleted from its N-terminus. The amino acid sequence of the recombinant human endostatin expressed in *E. coli* is shown in SEQ ID NO.2, but sometimes the N-terminal Met will be deleted after expression, and when this happens, the resultant recombinant human endostatin will have the sequence as shown in SEQ ID NO.1.

A mutant of endostatin refers to an endostatin molecule obtained by introducing amino acid replacement, deletion, or addition. An endostatin fragment refers to a part of the sequence of SEQ ID NO.1 or SEQ ID NO.2. The fragments may be obtained through, among others, enzymatic cleavage, genetic engineering expression, or polypeptide synthesis. For instance, the fragment with the sequence of SEQ ID NO.3, SEQ ID NO.4, or SEQ ID NO.5 was reported to have the activity to inhibit solid tumor (Cattaneo MG et al. Experimental Cell Research 283 (2003) 230 - 236; Tjin Tham Sjin RM et al. Cancer Research (2005) 65(9) 3656-3663). An isomer of endostatin refers to a molecule with the same amino acid sequence as the wild-type endostatin but with different conformations, including difference in the secondary or tertiary protein structure, or changed optical activity in regional amino acids. The isomer may be a naturally occurring mutant or obtained via artificial design. A derivative of endostatin refers to a product generated by modification of the wild-type endostatin. Modification refers to covalently attaching one or more small molecules, such as phosphoric acid molecules or carbohydrate molecules, or oligopeptide with less than 20 amino acids, to the protein at any amino acid of the protein. In a preferred embodiment, a 9-amino acid peptide chain containing a His-tag (MGGSHHHHH) is added to the N-terminus of human endostatin, and this derived endostatin has the sequence of SEQ ID NO.6 (Luo Yongzhang et al. US2004110671). When expressed in *E.coli,* its N-terminal Met sometimes will be deleted, and when this happens, the resultant N-terminal Met deleted derivative of recombinant human endostatin will have the sequence of SEQ ID NO.7. The combination of the active mutants, fragments, isomers or derivatives of endostatin means that the product simultaneously has two or more of the modifications mentioned above, for example without limitation, a mutant of a fragment or a modified mutant, et al.

### Examples

### Example 1: Coupling of 20 kDa PEG to the N-terminus of recombinant human endostatin

The recombinant human endostatins used in this example include recombinant human endostatins have the sequence of SEQ ID NO.2 and SEQ ID NO.6. Specifically, the recombinant human endostatin (Protgen Ltd.) was dialyzed into 30 mM sodium acetate solution, pH 5.5. Protein concentration was determined by measuring absorbance at 280 nm using UV spectrophotometer (Agilent Technologies), and then was adjusted to 2 mg/ml. A 20 kDa PEG (mPEG-ButyrALD 20 kDa, Nektar) which specifically modifies the N-terminus of protein was used for covalently coupling. Specifically, 80 mg of this 20 kDa PEG (solid) was added to 20 ml protein solution (containing 40 mg protein), and the mixed solution was stirred at room temperature until PEG solid dissolved completely and the molar ratio of PEG and endostatin was 2:1. CH₃BNNa (Sigma) was added as reductant to reach a final concentration of 20 mM, and finally the pH value of the solution was adjusted to 5. After resting at room temperature for 10 hours, more than 80% of endostatin was modified with mono-PEGylation, which means one PEG molecule was covalently linked to one endostatin molecule at N-terminal α-amino group of endostatin. Only a small amount of endostatin was non-specifically modified at multi-sites. The reaction solution can be purified directly through column chromatography. Electrophoresis analysis of solutions before and after the reaction is shown in Figures 7 and 8.

### Example 2: Purification of recombinant human endostatin modified with 20 kDa PEG at N-terminus through cation-exchange SP column

The recombinant human endostatins used in this example include recombinant human endostatins have the sequence of SEQ ID NO.2 and SEQ ID NO.6. Specifically, the human endostatin modified with 20 kDa PEG was purified through SP chromatography column (Amersham). The pH value of mixed solution was adjusted to 6 after reaction. The sample was loaded onto the column pre-equilibrated by a buffer containing 10 mM phosphate, pH 6.0. After loading the sample, gradient elution was performed with buffer containing 10 mM phosphate, 1 M NaCl, pH 6.0. The peak of PEG which did not involved in the reaction appeared during penetration and washing due to its minimal charge. The elution peaks appeared in the following order: multi-modified endostatin, mono-modified endostatin, and non-modified endostatin. Different fractions can be collected according to absorbance at 280 nm. The result of purification is shown in Figures 9 and 10.

### Example 3: Coupling of 40 kDa PEG to N-terminus of the recombinant human endostatin

The recombinant human endostatins used in this example include recombinant human endostatins have the sequence of SEQ ID NO.2 and SEQ ID NO.6. Specifically, the recombinant human endostatin was dialyzed into 30 mM sodium acetate solution, pH 5.5. The protein concentration was measured by UV spectrophotometer at 280 nm wavelength, and then was adjusted to 2.5 mg/ml. A 40 kDa PEG (mPEG-ButyrALD 40 kDa, Nektar) which specifically modifies the N-terminus of protein was used for covalently coupling. Specifically, 160 mg of this 40 kDa PEG (solid) was added to 20 ml protein solution (containing 40 mg protein), the solution was stirred at room temperature until PEG completely dissolved and the molar ratio of PEG and endostatin was 2:1. After that, CH₃BNNa (Sigma) was added as a reductant to a concentration of 20 mM, and finally, the pH value of solution was adjusted to 5. After resting at room temperature for 10 hours, more than 80% of the endostatin was modified by mono-PEGylation, i.e. one molecule of PEG was covalently coupled to one endostatin molecule at the N-terminal α-amino group of the protein. Only a small amount of endostatin was nonspecifically modified at multiple sites. The reaction solution can be purified directly through column chromatography.

### Example 4: Purification of recombinant human endostatin modified with 40 kDa PEG at N-terminus through cation-exchange SP column

The recombinant human endostatins used in this example include recombinant human endostatins have the sequence of SEQ ID NO.2 and SEQ ID NO.6. Specifically, the human endostatin modified with 40 kDa PEG was purified through SP chromatography column (Amersham). The pH value of mixed solution was adjusted to 6 after reaction. The sample was loaded onto the column pre-equilabrated by a buffer containing 10 mM phosphate, pH 6.0. After loading the sample, gradient elution was performed with buffer containing 10 mM phosphate, 1 M NaCl, pH 6.0. The peak of PEG which did not involved in the reaction appeared during penetration and washing due to its minimal charge. The elution peaks appeared in the following order: multi-modified endostatin, mono-modified endostatin, and non-modified endostatin. Different fractions can be collected according to absorbance at 280 nm. The result of purification of recombinant human endostatin having the sequence of SEQ ID NO.2 is shown in Figure 11. The result of purification of recombinant human endostatin having the sequence of SEQ ID NO.6 is similar to that of SEQ ID NO.2 (data not shown).

### Example 5: Prolonged efficacy of the recombinant human endostatin with 20 kDa PEG covalently coupled to its N-terminus in the blood

The recombinant human endostatins used in this example include recombinant human endostatins have the sequence of SEQ ID NO.2 and SEQ ID NO.6. The prolonged efficacy of the recombinant human endostatin with PEG modification was evaluated by measuring the *in vivo* half-life of the protein recombinant human endostatin with or without PEG modification, respectively. Six healthy Kunming mice (about 25g) (Vitalriver Experimental Animal Center) were divided into two groups, 3 mice per group. Then the mice were given an injection via tail vein of recombinant human endostatin (Protgen LTD.) either unmodified or N-terminus modified with 20 kDa PEG, respectively, in a dose of 15 mg/kg body weight. And then, blood samples were taken from tail vein at 2, 10, 30 minutes, 1, 2, 4, 8, 16, 24, 36, 48, and 72 hours. Plasma was then collected and the concentration of endostatin with or without PEG modification was measured by sandwich ELISA, respectively. As revealed by the pharmacokinetic analysis, upon modification with 20kDa PEG, the half-life of recombinant human endostatin with the sequence of SEQ ID NO.2 was increased from an average of 4.8 hours to an average of 16 hours in mice, and the half-life of recombinant human endostatin with the sequence of SEQ ID NO.6 was increased from an average of 7.5 hours to an average of 16 hours in mice. These results demonstrated that coupling of high molecular weight PEG to the recombinant human endostatin can effectively increase the *in vivo* half-life of the endostatin and achieve a prolonged efficacy.

### Example 6: The activity of recombinant human endostatin with 20 kDa PEG coupled to its N-terminus to inhibit the migration of vascular endothelial cells

The recombinant human endostatins used in this example include recombinant human endostatins have the sequence of SEQ ID NO.2 and SEQ ID NO.6. Specifically, the human microvascular endothelial cells (HMEC) were cultured in the DMEM medium (Hyclone) containing 10% serum to exponential phase. Then the cells were left starving for 12 hours. After digesting with trypsin (Sigma), cells were added into plates at 10000 cells per well for measurement of cell migration. Conditions for migration assay included DMEM medium, 1% serum, and 10 ng/ml bFGF (Sigma) and antibiotics (streptomycin and ampicillin, both at 10 µg/ml, Sigma). For the treatment groups, endostatin with or without modification was added to the cells at 30 µg/ml. After culturing for 8 hours at 37 °C, the cells in the plates were fixed with 1% glutaraldehyde and then the upper layer of cells on the membranes, which did not migrate, were removed, and the lower layer of cells were stained with hematoxylin. The cell number was counted under microscope (Olympus). Cells in three different fields on one plate were counted and then the inhibition rate was calculated. The results revealed that the inhibition rate on cells migration was 42% for recombinant human endostatin with the sequence of SEQ ID NO.2, while the inhibition rate on cells migration was 90% for modified endostatin. The inhibition rate on cells migration was 45% for the recombinant human endostatin of the sequence of SEQ ID NO.6, while the inhibition rate was 90% for modified endostatin. The above results demonstrate that the modification of endostatin with PEG will not only maintain but greatly enhance its biological activity. The experimental results are shown in Figure 12.

### Example 7: Activity of the recombinant human endostatin with 20 kDa PEG coupled to its N-terminus in the treatment of mice tumor model

The recombinant human endostatins used in this example include recombinant human endostatins have the sequence of SEQ ID NO.2 and SEQ ID NO.6. Experiments were performed to observe the *in vivo* inhibitory activity of endostatin modified by 20 kDa PEG on mice S180 tumor. Kunming mice of about 20 g were used in the experiment. Mice were inoculated at axillary with S180 sarcoma cells (China cell bank QK10952), 2 x 10⁶ cells per mouse. The mice were then randomly grouped on the following day, eight mice per group, including the negative control group (normal saline), the positive control group (endostatin, 1.5 mg/kg body weight, daily), three treatment groups (endostatin modified with PEG, 1.5 mg/kg body weight, daily, every other day, or every three days, respectively). The treatment was administered by way of tail vein injection. Administration lasted for 10 days. The mice were sacrificed on day 11, the weight of tumor was measured, and the efficacy was evaluated by tumor inhibition rate calculated as follows: tumor inhibition rate = (tumor weight of negative control group - tumor weight of treatment group)/tumor weight of negative control group × 100%. For the recombinant human endostatin with the sequence of SEQ ID NO.2, the experimental results revealed that the tumor inhibition rate of the positive control group was 34%, whereas the tumor inhibition rates were 47%, 53% and 40% for the groups treated daily, every other day and every three days, respectively. For the recombinant human endostatin having the sequence of SEQ ID NO.6 with N-terminal additional amino acids, the experimental results revealed that the tumor inhibition rate of positive control group was 40%, whereas the tumor inhibition rates were 45%, 57% and 50% for the groups treated daily, every other day and every three days, respectively. The results demonstrate that the recombinant human endostatin with modification indeed has the antitumor activity in vivo, and since it has a slow *in vivo* metabolic rate, the activity of the modified recombinant human endostatin is superior to that of the unmodified recombinant human endostatin. Furthermore, still it can maintain its superior efficacy as compared with the unmodified recombinant human endostatin even administered at a prolonged intervel. The experimental results are shown in Figure 13.

### Example 8: Modification of ε-amino group on the side chain of lysine residue of the recombinant human endostatin with 20 kDa PEG

The recombinant human endostatins used in this example include recombinant human endostatins have the sequence of SEQ ID NO.2 and SEQ ID NO.6. Specifically, the recombinant human endostatin is dialyzed into 30 mM phosphate buffer solution, pH 7.8. The protein concentration is measured by UV spectrophotometer at 280 nm wavelength, and then is adjusted to 2 mg/ml. A 20 kDa PEG (mPEG-SPA 20 kDa, Nektar) which modifies the ε-amino group on the side chain of lysine residue is added and the molar ratio of PEG and the protein is 8:1. The recombinant human endostatin can be modified quickly, and after placing at room temperature for 30 minutes, 80% of the endostatin is modified. The amount of modification product increases with the time of reaction, but the reaction speed slows down after 1 hour. The products are mainly mono-PEGylated and multi-PEGylated endostatin. The reaction solution can be purified directly through column chromatography.

### Example 9: Molecular sieve purification of recombinant human endostatin with 20 kDa PEG modification on ε-amino group on the side chain of lysine residue

The recombinant human endostatins used in this example include recombinant human endostatins have the sequence of SEQ ID NO.2 and SEQ ID NO.6. The endostatin modified by 20 kDa PEG was purified by molecular sieve. After reaction the pH value of the mixture was adjusted to 7.4. The sample was loaded onto the column pre-equilabrated by a buffer containing 20 mM phosphate, 100 mM NaCl, pH 7.4. Different fractions can be collected according to the absorbance at 280 nm using a UV spectrophotometer. The purification result of the modified endostatin with the sequence of SEQ ID NO.6 is shown in the Figure 14.

### Example 10: Ion-exchange chromatography purification of the recombinant human endostatin with 20 kDa PEG modification on the ε-amino group on the side chain of lysine residue

The recombinant human endostatins used in this example include recombinant human endostatins have the sequence of SEQ ID NO.2 and SEQ ID NO.6. The 20 kDa PEG-endostatin conjugate was purified by CM chromatography column. After reaction, the pH value of the mixture was adjusted to 6.5. The sample was loaded onto the column pre-equilabrated by a buffer containing 10 mM phosphate, pH 6.5. After loading the sample, gradient elution was performed with buffer containing 10 mM phosphate, 1 M NaCl, pH 6.5. The peak of PEG which did not involved in the reaction appeared during penetration and washing due to its minimal charge. The elution peaks appeared in the following order: multi-modified endostatin, mono-modified endostatin, and non-modified endostatin. Different fractions can be collected according to absorbance at 280 nm. The result of purification of the modification product of endostatin having a sequence of SEQ ID NO.6 is shown in Figure 15.

### Example 11: Activity of the recombinant human endostatin with N-terminal modification with 20 kDa PEG in the treatment of mice tumor model

The recombinant human endostatin used in this example has the sequence of SEQ ID NO.6. The experiment was to observe the *in vivo* inhibitory activity of endostatin modified with 20 kDa PEG (mPEG-ButyrALD 20 kDa, Nektar) administered at increasing intervals on the growth of S180 tumor in mice. Kunming mice (20g) were used in the experiment. The mice were inoculated at axillary with S180 sarcoma cells (China cell bank QK10952), 2 x 10⁶ cells per mouse. The mice were then randomly grouped on the following day, eight mice per group, including the negative control group (normal saline), the positive control group (endostatin, 1.5 mg/kg body weight, administered every five days), four treatment groups (endostatin modified with PEG, 1.5 mg/kg body weight, administered every five days, every seven days, every ten days, or every fourteen days, respectively). The treatment was administered by way of tail vein injection. Administration lasted for 14 days. The mice were sacrificed on day 15, the weight of tumor was measured, and the efficacy of treatment was evaluated by tumor inhibition rate calculated as follows: tumor inhibition rate = (tumor weight of negative control group - tumor weight of treatment group)/tumor weight of negative control group × 100%. For the recombinant human endostatin having the sequence of SEQ ID NO.6 with N-terminal additional amino acids, the experimental results revealed that the tumor inhibition rate of the positive control group was 34%, whereas the tumor inhibition rates were 56.4%, 37.1% and 31.8%, and 0% for the groups treated every five days, every seven days, every ten days, or every fourteen days, respectively. This suggests that the modified recombinant human endostatin can still exhibit *in vivo* inhibitory activity on tumor growth even administered at an interval as long as 10 days.

## Claims

1. A conjugate formed by coupling one polyethylene glycol molecule to one endostatin molecule, wherein the polyethylene glycol molecule is coupled to endostatin at the N-terminal α-amino group of endostatin, and the polyethylene glycol has an average molecular weight from 5 kDa to 40 kDa, and wherein the conjugate has enhanced biological activities as compared to the corresponding non-modified endostatin molecule.

2. The conjugate of claim 1, wherein said polyethylene glycol molecule is either linear or branched.

3. The conjugate of claim 1 or 2, wherein the polyethylene glycol has an average molecular weight of 20 kDa or 40 kDa.

4. The conjugate of any one of claims 1-3, wherein the polyethylene glycol is selected from the group consisting of monomethoxy polyethylene glycol, monohydroxyl polyethylene glycol and derivatives thereof.

5. The conjugate of any one of claims 1-4, wherein the endostatin is of mammal origin, particularly of human or murine origin.

6. The conjugate of claim 5, wherein the endostatin includes an active fragment, mutant, derivative, or isomer of endostatin.

7. The conjugate of claim 6, wherein the endostatin has a sequence selected from the group consisting of SEQ ID NO.1, SEQ ID NO.2, SEQ ID NO.3, SEQ ID NO.4, SEQ ID NO.5, SEQ ID NO.6 or SEQ ID NO.7.

8. A sustained-release formulation formed by a conjugate of any one of claims 1-7 with a bio-compatible substance, wherein the sustained-release formulation is in a form selected from the group consisting of microcapsule, hydrogel, microsphere, micro-osmotic pump and liposome.

9. A pharmaceutical composition comprising a conjugate of any one of claims 1-7 or a sustained-release formulation of claim 8 and a pharmaceutically acceptable carrier.

10. The pharmaceutical composition of claim 9, wherein the pharmaceutically acceptable carrier is an aqueous pH buffer comprising a buffer solution of phosphate, a buffer solution of citrate; ascorbic acid; serum albumin, glutin, immunoglobulin; polyvinylpyrrolidone; glycine, glutamine, asparagine, arginine, lysine; monosaccharide, disaccharide, glucose, mannose, dextrin; EDTA, sorbitol; sodium ion; TWEEN®, PEG, and/or PLURONICS®.

11. Use of a conjugate of any one of claims 1-7, a sustained-release formulation of claim 8, or a pharmaceutical composition of claim 9 or 10 in the preparation of a medicament for the treatment of tumor.

12. The use of claim 11, where the tumor is selected from the group consisting of lung cancer, neuroendocrine tumor, colon cancer, bone cancer, liver cancer, gastric cancer, pancreatic cancer, oral cancer, breast cancer, prostate cancer, lymphoma, esophagus cancer, nasopharyngeal carcinoma, cervical cancer, sarcoma, renal cell carcinoma, biliary cancer, and malignant melanoma.

13. A method for prolonging the *in vivo* half-life and enhancing the biological activities of endostatin, comprising forming a conjugate by coupling one polyethylene glycol molecule to one endostatin molecule, wherein the coupling site is the N-terminal α-amino group of endostatin, and the polyethylene glycol has an average molecular weight from 5 kDa to 40 kDa.

## Patentansprüche

1. Konjugat, das durch Koppeln eines Polyethylenglycolmoleküls an ein Endostatinmolekül gebildet ist, wobei das Polyethylenglycolmolekül an der N-terminalen α-Aminogruppe des Endostatins an Endostatin gekoppelt ist, und das Polyethylenglycolmolekül ein mittleres Molekulargewicht von 5 kDa bis 40 kDa aufweist, und wobei das Konjugat, verglichen mit dem entsprechenden nicht modifizierten Endostatinmolekül, verbesserte biologische Aktivitäten aufweist.

2. Konjugat nach Anspruch 1, wobei das Polyethylenglycolmolekül entweder linear oder verzweigt ist.

3. Konjugat nach Anspruch 1 oder 2, wobei das Polyethylenglycol ein mittleres Molekulargewicht von 20 kDa oder 40 kDa aufweist.

4. Konjugat nach einem der Ansprüche 1 bis 3, wobei das Polyethylenglycol aus der Gruppe bestehend aus Monomethoxypolyethylenglycol, Monohydroxylpolyethylenglycol und Derivaten davon ausgewählt ist.

5. Konjugat nach einem der Ansprüche 1 bis 4, wobei das Endostatin von Säugetierherkunft, insbesondere von menschlicher oder muriner Herkunft ist.

6. Konjugat nach Anspruch 5, wobei das Endostatin ein aktives Fragment, eine Mutante, ein Derivat oder ein Isomer von Endostatin beinhaltet.

7. Konjugat nach Anspruch 6, wobei das Endostatin eine Sequenz ausgewählt aus der Gruppe bestehend aus SEQ ID NO.1, SEQ ID NO. 2, SEQ ID NO.3, SEQ ID NO.4, SEQ ID NO.5, SEQ ID NO.6 oder SEQ ID NO.7. aufweist.

8. Formulierung mit Freisetzung bei konstanter Geschwindigkeit, die durch ein Konjugat nach einem der Ansprüche 1 bis 7 mit einer biokompatiblen Substanz gebildet wird, wobei die Formulierung mit Freisetzung bei konstanter Geschwindigkeit in einer Form ausgewählt aus der Gruppe bestehend aus einer Mikrokapsel, einem Hydrogel, einer Mikrosphäre, einer mikroosmotischen Pumpe und einem Liposom ist.

9. Pharmazeutische Zusammensetzung, die ein Konjugat nach einem der Ansprüche 1 bis 7 oder eine Formulierung mit Freisetzung bei konstanter Geschwindigkeit nach Anspruch 8 und einen pharmazeutisch akzeptablen Träger umfasst.

10. Pharmazeutische Zusammensetzung nach Anspruch 9, wobei der pharmazeutisch akzeptable Träger ein wässriger pH-Puffer ist, der eine Phosphat-Pufferlösung, eine Citrat-Pufferlösung; Ascorbinsäure; Serumalbumin, Glutin, ein Immunoglobulin; Polyvinylpyrrolidon; Glycin, Glutamin, Asparagin, Arginin, Lysin; ein Monosaccharid, ein Disaccharid, Glukose, Mannose, Dextrin; EDTA, Sorbitol; ein Natriumion; TWEEN®, PEG, und/oder PLURONICS® umfasst.

11. Verwendung eines Konjugats nach einem der Ansprüche 1 bis 7, einer Formulierung mit Freisetzung bei konstanter Geschwindigkeit nach Anspruch 8 oder einer pharmazeutischen Zusammensetzung nach Anspruch 9 oder 10 bei der Zubereitung eines Medikaments für die Behandlung eines Tumors.

12. Verwendung nach Anspruch 11, wobei der Tumor aus der Gruppe bestehend aus Lungenkrebs, einem neuroendokrinen Tumor, Darmkrebs, Knochenkrebs, Leberkrebs, einem Magenkarzinom, Bauchspeicheldrüsenkrebs, Mundkrebs, Brustkrebs, Prostatakrebs, einem Lymphom, Speiseröhrenkrebs, ein Nasen-Rachenraum-Karzinom, Gebärmutterhalskrebs, ein Sarkom, ein Nierenzellenkarzinom, Blasenkrebs und malignen Melanomen ausgewählt ist.

13. Verfahren für die Verlängerung der *in vivo*-Halbwertszeit und der Erhöhung der biologischen Aktivitäten von Endostatin, umfassend die Bildung eines Konjugats durch Koppeln eines Polyethylenglykolmoleküls an ein Endostatinmolekül, wobei die Kopplungsstelle die N-terminale α-Aminogruppe von Endostatin ist, und das Polyethylenglykol ein mittleres Molekulargewicht von 5 kDa bis 40 kDa aufweist.

## Revendications

1. Un conjugué formé par couplage d'une molécule de polyéthylène glycol à une molécule d'endostatine, dans lequel la molécule de polyéthylène glycol est couplée à l'endostatine sur le groupe α-amino N-terminal de l'endostatine et le polyéthylène glycol a une masse moléculaire moyenne de 5 à 40 kDa, et dans lequel le conjugué présente des propriétés biologiques améliorées par rapport à la molécule d'endostatine non modifiée correspondante.

2. Le conjugué selon la revendication 1, dans lequel le polyéthylèneglycol est linéaire ou ramifiée.

3. Le conjugué selon la revendication 1 ou 2, dans lequel le polyéthylèneglycol a une masse moléculaire moyenne de 20 ou 40 kDa.

4. Le conjugué selon l'une quelconque des revendications 1 à 3, dans lequel ledit polyéthylène glycol est choisi dans le groupe comprenant le monométhoxy polyéthylène glycol, le monohydroxy polyéthylèneglycol et leurs dérivés.

5. Le conjugué selon l'une quelconque des revendications 1 à 4, dans lequel ladite endostatine est d'origine mammifère, plus particulièrement est d'origine humaine ou murine.

6. Le conjugué selon la revendication 5, dans lequel l'endostatine inclut un fragment actif, un mutant, un dérivé ou un isomère d'endostatine.

7. Le conjugué selon la revendication 6, dans lequel l'endostatine présente une séquence choisie dans le groupe formé par les SEQ ID NO.1, SEQ ID NO.2, SEQ ID NO.3, SEQ ID NO.4, SEQ ID NO.5, SEQ ID NO.6, SEQ ID NO.7.

8. Une formulation à libération prolongée formée d'un conjugué selon une des revendications 1 à 7, avec une substance biocompatible, laquelle formulation à libération prolongée est sous une forme choisie dans le groupe consistant en les microcapsules, hydrogel, microsphères, pompes micro-osmotiques ou liposomes.

9. Une composition pharmaceutique comprenant un conjugué selon l'une quelconque des revendications 1 à 7 ou une formulation à libération prolongée selon la revendication 8 et un support pharmaceutiquement acceptable.

10. La composition pharmaceutique selon la revendication 9, dans laquelle le support pharmaceutiquement acceptable est un tampon pH aqueux comprenant une solution tampon de phosphate, une solution tampon de citrate; l'acide ascorbique; l'albumine sérique, la glutine, l'immunoglobuline; la polyvinylpyrrolidone; la glycine, la glutamine, l'asparagine, l'arginine, la lysine; le monosaccharide, le disaccharide, le glucose, le mannose, la dextrine; EDTA, sorbitol; ion sodium; TWEEN®, PEG, et/ou PLURONICS®.

11. L'utilisation d'un conjugué selon l'une quelconque des revendications 1 à 7 ou d'une formulation à libération prolongée selon la revendication 8 ou une composition pharmaceutique selon la revendication 9 ou 10, pour la préparation d'un médicament pour le traitement de tumeur.

12. L'utilisation selon la revendication 11, dans laquelle la tumeur est choisie dans le groupe consistant en le cancer du poumon, la tumeur neuroendocrine, le cancer du côlon, le cancer des os, le cancer du foie, le cancer gastrique, le cancer du pancréas, le cancer de la bouche, le cancer du sein, le cancer de la prostate, le lymphome, le cancer de l'oesophage, le carcinome nasopharyngé, le cancer du col de l'utérus, le sarcome, le carcinome des cellules rénales, le cancer biliaire et le mélanome malin.

13. Un procédé pour prolonger la demi-durée de vie in vivo et d'améliorer les activités biologiques de l'endostatine, comprenant la formation d'un conjugué par couplage d'une molécule de polyéthylène glycol à une molécule d'endostatine, dans lequel le site de couplage est le groupe α-amino N-terminal de l'endostatine et le polyéthylène glycol a une masse moléculaire moyenne de 5 à 40 kDa.
